# EUROPEAN PATENT SPECIFICATION

(11) **EP 1 359 902 B1**
(45) Date of publication and mention of the grant of the patent: **08.08.2007**
(21) Application number: 02712031.0
(22) Date of filing: 05.02.2002
(51) Int. Cl.: A61K 9/14, A61K 9/72, A61K 31/565, A61K 31/167

(54) **BIMODAL DRY POWDER FORMULATION FOR INHALATION**
BIMODALE TROCKENPULVERZUSAMMENSETZUNG ZUR INHALATION
COMPOSITION BIMODALE DE POUDRE SECHE POUR L'INHALATION

(30) Priority: 06.02.2001 GB 0102902; 12.04.2001 GB 0109215
(43) Date of publication of application: 12.11.2003
(73) Proprietor: Innovata Biomed Limited, St. Albans AL1 3HW (GB)
(72) Inventor: SANDERS, Mark, Innovata Biomed Limited, St Albans AL1 3HW (GB)
(74) Representative: Jones, Stephen Anthony
(86) International application number: PCT/GB2002/000480
(87) International publication number: WO 2002/062317

(56) References cited:
- WO-A-95/00128
- WO-A-98/31352
- FR-A- 2 584 604
- GERRITY T R: "PATHOPHYSIOLOGICAL AND DISEASE CONSTRAINTS ON AEROSOL DELIVERY" RESPIRATORY DRUG DELIVERY, XX, XX, 1990, pages 1-38, XP008006563

## Description

This invention relates to a novel medicament, novel formulations comprising the medicament and novel methods of treatment.

UK Patent No. 1242211 describes pharmaceutical combination products comprising sodium cromoglycate and isoprenaline sulphate as active ingredients and wherein the particle size of each of the active ingredients is in the range of from 1 to 10 µm.

European Patent No. 0 663 815 describes an inhalation powder which comprises a micronised active substance and a pharmaceutically acceptable excipient wherein the excipient contains a coarse fraction having an average particle size of 20 µm or more and a fine fraction with an average particle size of 10µm or less.

International Patent Application No. WO 01/60341, which is an intervening publication, describes a powder formulation, for administration by inhalation, which comprises a mixture of an active substance which has a particle size distribution of 0.5 to 10µm and an excipient which has a particle size distribution of from 15 to 500µm.

International Patent Application No. WO 01/51030, which is a further intervening publication, describes a 'bimodal' formulation which comprises fine particles for delivery to the lung and coarse particles for delivery to the GI tract. However, such bimodal compositions do not offer any particular improvement in inhalation therapies *per se.*

French Patent Application No. 2584604 describes a new pharmaceutical dosage form for oral, enteral, administration comprising a fine powder (<100 µm) and coarse crystals (500-700 µm). However, such a composition is not suitable for administration, for example, by inhalation.

International Patent Application No. WO98/31352 describes an inhalation therapy comprising a mixture of, for example, micronised formoterol fumarate dihydrate (MMD <3 µm) and micronised budesonide (particle size <3 µm).

We have now surprisingly found that the administration of a combination of active ingredients each of which has different particle sizes may be advantageous. In particular, we have found that a combination therapy comprising a coarse active ingredient and a fine active ingredient is especially useful in the treatment of respiratory disorders.

Thus, according to the invention we provide a bimodal pharmaceutical composition comprising effective amounts of (i) a particulate coarse active ingredient and (ii) a particulate fine active ingredient, characterised in that the coarse ingredient possesses a greater mass median aerodynamic diameter (MMAD) than the fine ingredient, the MMAD of the coarse active ingredient being 4-20µm and the MMAD of the fine active ingredient being 1-4µm, and wherein the coarse ingredient comprises an agent which is active in the central/upper airways of a patient.

Particle size is commonly defined using mass median aerodynamic diameter (MMAD). Thus, hereinafter any reference to specific particle sizes should be construed as meaning MMAD unless otherwise defined as, for example, aerodynamic diameter. Although the sizes of the coarse and fine particles may vary, it should be understood that the coarse ingredient possesses a greater MMAD than the fine ingredient. That is, the majority, by mass, of the particles in the coarse ingredient possess greater aerodynamic diameters than the majority of particles of the fine ingredient.

Provided that the composition is bimodal as hereinbefore described, the aerodynamic particle size of the coarse ingredient may be from 4 to 20 µm, preferably from 4 to 12 µm e.g. 6 µm. That is, at least 50% w of the particles have an aerodynamic particle diameter 6 pm. The aerodynamic particle size of the fine ingredient may be from 1 to 4 µm, e.g. 1 µm. That is, at least 50% w/w of the particles have an aerodynamic particle size of 1 µm,

The coarse ingredient comprises an agent which is active in the central/upper airways of a patient, whilst the fine ingredient may comprise an agent which is active in the lung periphery.

In one embodiment of the invention the composition of the invention may be utilised in the treatment of any disorders known to be affected by corticosteroids and/or β-agonists. Thus for example the pharmaceutical composition can be useful in the treatment of non-endrocrine disorders including allergy, anaphylaxis, arteritis, collagenosis, blood disorders, cardiovascular disorders, gastro-intestinal disorders, hypercalcaemia, muscular disorders, ocular disorders, renal disorders, respiratory disease, rheumatic disorders and skin disorders.

In a preferred embodiment of the invention the pharmaceutical composition is useful, *inter alia,* in the treatment of respiratory disorders. In such a composition the fine ingredient preferentially may comprise an anti-inflammatory medicament, such as a corticosteroid, whilst the coarse ingredient may comprise a bronchodilator.

The coarse ingredient comprises a medicament which is active in the central/upper airways of a patient, such as a bronchodilator, a mucolytic agent, an antibiotic, etc.

The bronchodilators used in the composition of the invention may be selected from, but are not limited to, β₂-agonists e.g. fenoterol, formoterol, pirbuterol, reproterol, rimiterol, salbutamol, salmeterol and terbutaline; non-selective beta-stimulants such as isoprenaline; xanthine bronchodilators, e.g. theophylline, aminophylline and choline theophyllinate; anticholinergics, e.g. ipratropium bromide; isomers and/or combinations thereof.

The corticosteroids used in the composition of the invention may be selected from, but are not limited to, beclomethasone dipropionate, fluticasone, budesonide, flunisolide, ciclesonide, triamcinolone, e.g. the acetonide, and mometasone; isomers and/or combinations thereof.

Specific combinations of medicaments which may be mentioned include combinations of steroids, such as, beclomethasone dipropionate and formoterol; beclomethasone dipropionate and salmeterol; fluticasone and formoterol; fluticasone and salmeterol; budesonide and formoterol; budesonide and salmeterol; flunisolide and formoterol; and flunisolide and salmeterol. It is also within the scope of this invention to include combinations of one or more of the aforementioned steroids with one or more of the aforementioned β₂-agonists.

The most preferred composition of the invention is one which comprises a combination of fluticasone, or a pharmaceutically acceptable ester thereof, e.g. the propionate ester, and formoterol, or a pharmaceutically acceptable salt thereof.

In the bronchodilator/corticosteroid combination composition it is preferable that the coarse ingredient comprises the bronchodilator and the fine ingredient comprises the corticosteroid.

Alternatively, the composition of the invention may deliver one or more systemically active medicaments, in which case the coarse ingredient may comprise, for example, a bronchodilator and the fine ingredient may comprise an active agent, such as an antibiotic or a large macromolecule. Examples of such large macromolecules include, but are not limited to polypeptides, such as, insulin, growth hormone, leuprolide, interferon, parathyroid hormone and the like; and analgesic compounds, such as morphine, M6G and fentanyl.

The fine ingredient and/or the coarse ingredient may, for example, also include an absorption enhancer.

Alternatively, or in addition, the coarse ingredient may also include a signalling agent, for example, a flavouring agent. The term flavouring agent should be construed so as to include sweetening agents. Any conventionally known flavouring agents may be used. Such flavouring agents include, but are not limited to, peppermint oil, menthol, sugar, aspartame, cyclamates and saccharin, and salts thereof, or any combination of the aforesaid.

Therefore, in this embodiment of the invention the coarse ingredient may comprise a signalling agent and an active ingredient which is active in the central/upper airways of a patient, whilst the fine ingredient may comprise an agent which is active in the lung periphery.

Thus according to a further feature of the invention we provide a pharmaceutical composition as hereinbefore described which comprises a fine ingredient comprising a first active ingredient and a coarse ingredient comprising a signalling agent and a second active ingredient.

When the coarse ingredient comprises a signalling agent and a second active ingredient, the signalling agent and the second active ingredient may comprise particles of substantially similar aerodynamic particle sizes.

Alternatively, the signalling agent may comprise particles which are substantially of greater aerodynamic particle size than the second active ingredient. Thus, although not essential, such compositions may optionally be in the form of a trimodal composition.

The preferred pharmaceutical composition of the invention is most advantageous in the treatment of respiratory disorders and especially asthma and chronic obstructive pulmonary disease (COPD).

In the treatment of respiratory and/or systemic disorders the pharmaceutical composition may be delivered to the respiratory tract. Thus, delivery to the respiratory tract may comprise buccal delivery, nasal delivery or delivery by inhalation. The preferred mode of delivery to the respiratory tract is by inhalation into the lungs. Thus, the pharmaceutical composition can be administered by way of an inhaler, e.g. a metered dose inhaler or a dry powder inhaler, an insufflator or nebuliser, or any other conventionally known methods of administering inhalable medicaments.

When administered by way of inhalation the pharmaceutical composition may be in the form of a pressurised aerosol. Thus, according to a further feature of the invention we provide a pharmaceutical formulation suitable for administration by way of a pressurised aerosol comprising a pharmaceutical composition as hereinbefore described in admixture with at least a suitable propellant and optionally with a surfactant or a mixture of surfactants. The propellant is preferably a non-CFC propellant, such as a hydrofluoroalkane (HFA). Any conventionally known HFA propellant may be used, including those disclosed in, for example, EP0372777, WO91/04011, WO91/11173, WO91/11495 and WO91/14422. However, the most preferred HFA is a fluoroalkane such as a fluoromethane or a fluoroethane or a mixture of fluoroalkanes. Such fluoroalkanes include, but are not limited to, trichlorofluoromethane, dichlorodifluoromethane, 1,2-dichlorotetrafluorethane, trichlorotrifluoroethane and chloropentafluoroethane. The most preferred is HFA 134 (1,1,1,2-tetrafluoroethane) or HFA 227. The amount of propellant present may vary, but generally the pharmaceutical composition to propellant ratio will be from 1 to 300 to 1 to 5. Mixtures of propellants may also be used, for example, a mixture of HFA 134 and HFA 227. The aerosol composition of the invention may be as a solution or a suspension of the active ingredient with a propellant.

The pressurised aerosol formulation of the invention may be administered in any conventionally known inhalation apparatus.

In another embodiment the pharmaceutical composition may be administered as a dry powder formulation. Thus, according to the invention we provide a pharmaceutical formulation suitable for administration by way of a dry powder inhaler comprising a pharmaceutical composition as hereinbefore described optionally in admixture with a suitable adjuvant, diluent or carrier. When the formulation does include an adjuvant, diluent or carrier, any conventionally used ingredients in dry powder formulations may be used, such as sugars, these include, but are not limited to, dextran, mannitol and lactose, e.g. α-lactose monohydrate. Preferably, the pharmaceutical composition to carrier ratio is from 0.01 : 1 to 50 : 1.

The dry powder formulation of the invention may be administered in any conventionally known inhalation apparatus.

In a dry powder inhaler the coarse ingredient and the fine ingredient may be administered simultaneously, sequentially or separately.

However, preferred apparatus are those commercially available as CLICKHALER which is described in International Patent Application No. WO 92/00771 and/or TECHNOHALER which is described in International Patent Application No. WO 93/16748.

Alternatively, the formulation may be administered by way of a conventional nebuliser. A suitable nebuliser formulation consists of a suspension of .a pharmaceutical composition of the invention in finely divided form in a sterile isotonic solvent. The suspension may be nebulised by an air jet, dropping onto an ultrasonic vibrating plate, forcing through small orifices or other known types of nebuliser, including unit-dose nebulisers, including those described by Dolovich, M., "New Propellant-free Technologies under Investigation", J. Aerosol Medicine, 1999; 12 (suppl 1: S9-S17, such as, Respimat (from Boehringer Ingelheim), AERx^{™} (from Aradigm), and AeroDose (from Aerogen).

For inhalation therapy the pharmaceutical composition is preferably micronised or reduced in size by other recognised mechanisms, such as spray drying, co-milling, etc.

The dosage of pharmaceutical composition administered to a patient may vary depending, *inter alia,* upon the nature and severity of the disorder being treated and the method of administration. For compositions administered by inhalation therapy, the amount of the pharmaceutical composition administered may vary, depending upon, *inter alia,* the nature of the pharmaceutical, the disorder to be treated, the mode of administration, etc. Thus, for example, when the pharmaceutical includes an antibiotic or when the mode of administration is, by nebuliser, then the dosage is preferably in the range of from 1 µg to 500 mg. This may be 1 µg to 500 mg per metered dose or actuation or, alternatively, 1 µg to 500 mg from a plurality of metered doses or actuations. Alternatively, especially when other modes of administration are used the dosage may be in the range of from 1 µg to 300mg, more preferably from 1 µg to 20 mg and especially from 1 µg to 5 mg.

In an especially preferred embodiment each metered dose or actuation of the inhaler will generally contain from 3 µg to 200 µg of a coarse fraction, e.g. a bronchodilator, preferably from 3 to 50 µg; and from 20 µg to 1,000 µg of a fine fraction, e.g. a corticosteroid, preferably from 20 to 500 µg. The frequency of administration of the pharmaceutical composition of the invention will vary, but most preferably, the pharmaceutical composition will be administered once or twice daily.

In the invention the coarse and fine ingredients may be administered simultaneously, sequentially or separately.

In a preferred embodiment the coarse ingredient is delivered to the central or upper airways of a patient and the fine ingredient is delivered to the lung periphery. In the most preferred embodiment the coarse and fine ingredients are delivered simultaneously as a single composition as hereinbefore described. Alternatively, particularly if the coarse ingredient comprises, for example, a bronchodilator, the coarse and fine ingredients may be delivered sequentially. Thus, for example, the method may comprise the administration of the coarse ingredient, followed by the sequential administration of the fine ingredient.

According to a further feature of the invention we provide the use of (i) a particulate coarse bronchodilator and (ii) a particulate fine anti-inflammatory agent in the manufacture of a medicament for the treatment of a respiratory disorder, characterised in that the coarse ingredient possesses a greater mass median aerodynamic diameter (MMAD) than the fine ingredient, the MMAD of the coarse active ingredient being 4-20µm and the MMAD of the fine active ingredient being 1-4µm, and wherein the coarse ingredient comprises an agent which is active in the central/upper airways of a patient.

In a further embodiment of the invention the fine ingredient may comprise a macromolecule as hereinbefore described, an antibiotic, a mucolytic agent, etc., optionally in combination with an absorption enhancer.

When a signalling agent is included, the signalling agent may be administered simultaneously, sequentially or separately with the active ingredients. Alternatively, the signalling agent may be delivered simultaneously with one or other of the coarse or fine ingredients, whilst being delivered separately or sequentially with the other of the coarse or fine ingredient. Since the signalling agent is itself preferentially comprised of substantially coarse particles, then in a preferred embodiment of the invention the signalling agent may be administered simultaneously with the coarse ingredient.

The respiratory disorder that may be treated using the composition of the invention may be COPD, and the fine and coarse ingredients may be a corticosteroid and a bronchodilator.

The anti-inflammatory agent and the bronchodilator may be administered as separate compositions, which may be administered simultaneously, sequentially or separately or as a single combination product. Each metered dose or actuation of the inhaler will generally contain from 3 µg to 50 µg of the bronchodilator and from 20 µg to 500 µg of the anti-inflammatory agent. The frequency of administration of the pharmaceutical composition of the invention will vary, but most preferably, the pharmaceutical composition will be administered once or twice daily in, for example, the treatment of asthma.

In a preferred embodiment, the method of treatment using the invention comprises the administration of a therapeutically effective amount of a corticosteroid and a bronchodilator as a pharmaceutical composition as hereinbefore described.

In a bronchodilator/corticosteroid combination therapy, the ratio of bronchodilator to corticosteroid in the composition according to the invention may vary, but is preferably within the range from 1: 0.4 to 1 : 167.

We further provide a process for the manufacture of a pharmaceutical composition as hereinbefore described which comprises mixing a coarse active ingredient with a fine active ingredient , and optionally at the same time or sequentially mixing a pharmaceutically acceptable adjuvant, diluent or carrier.

A variety of medicaments may be administered simultaneously, sequentially or separately with the composition of the invention. Such medicaments are generally antibiotics, bronchodilators or other anti-asthma drugs. Such medicaments include, but are not limited to β₂-agonists, e.g. fenoterol, formoterol, pirbuterol, reproterol, rimiterol, salbutamol, salmeterol and terbutaline; non-selective beta-stimulants such as isoprenaline; xanthine bronchodilators, e.g. theophylline, aminophylline and choline theophyllinate; anticholinergics, e.g. ipratropium bromide; mast cell stabilisers, e.g. sodium cromoglycate and ketotifen; bronchial anti-inflammatory agents, e.g. nedocromil sodium; and steroids, e.g. beclomethasone dipropionate, fluticasone, budesonide, ciclesonide, triamcinolone, e.g. the acetonide, and flunisolide; and combinations thereof.

The invention will now be illustrated but shall not be limited to the following example.

### Example 1

### Formulation

A bimodal dry powder inhalation formulation was prepared comprising:
as coarse ingredient- 6 µg formoterol fumarate with particle size 5-6 µm
as fine ingredient- 100 µg fluticasone propionate with particle size 2 - 3 µm

## Claims

1. A bimodal pharmaceutical composition comprising effective amounts of (i) a particulate coarse active ingredient and (ii) a particulate fine active ingredient, **characterised in that** the coarse ingredient possesses a greater mass median aerodynamic diameter (MMAD) than the fine ingredient, the MMAD of the coarse active ingredient being 4-20µm and the MMAD of the fine active ingredient being 1-4µm, and wherein the coarse ingredient comprises an agent which is active in the central/upper airways of a patient.

2. A bimodal pharmaceutical composition according to claim 1 **characterised in that** the fine active ingredient comprises an agent which is active in the lung periphery.

3. A bimodal pharmaceutical composition according to any one of claims 1 and 2 **characterised in that** the aerodynamic particle size of the coarse ingredient is from 4 to 20µm.

4. A bimodal pharmaceutical composition according to Claim 3 **characterised in that** at least 50% w/w of the coarse particles have an aerodynamic particle size of from 4 to 12µm.

5. A bimodal pharmaceutical composition according to Claim 4 **characterised in that** at least 50% w/w of the coarse particles have an aerodynamic particle size of 6 µm.

6. A bimodal pharmaceutical composition according to any preceding claim **characterised in that** the aerodynamic particle size of the fine ingredient is from 1 to 4 µm.

7. A bimodal pharmaceutical composition according to Claim 6 **characterised in that** at least 50% w/w of the fine particles have an aerodynamic particle size of from 1 to 4 µm.

8. A bimodal pharmaceutical composition according to Claim 7 **characterised in that** at least 50% w/w of the fine particles have an aerodynamic particle size of I µm.

9. A bimodal pharmaceutical composition according to any one of the preceding claims **characterised in that** the pharmaceutical composition is suitable for the treatment of one or more disorders selected from allergy, anaphylaxis, arteritis, collagenosis, blood disorders, cardiovascular disorders, gastro-intestinal disorders, hypercalcaemia, muscular disorders, ocular disorders, renal disorders, respiratory disease, rheumatic disorders and skin disorders.

10. A bimodal composition according to any one of the previous claims **characterised in that** the composition includes a signalling agent.

11. A bimodal composition according to claim 10 **characterised in that** the signalling agent is comprised in the coarse ingredient.

12. A bimodal composition according to claim 11 **characterised in that** the coarse signalling agent creates a trimodal composition.

13. A bimodal pharmaceutical composition according to Claim 9 **characterised in that** the pharmaceutical composition is suitable for the treatment of respiratory disorders.

14. A bimodal pharmaceutical composition according to claim 13 **characterised in that** the fine ingredient comprises an anti-inflammatory agent.

15. A bimodal pharmaceutical composition according to Claim 13 or claim 14 **characterised in that** the coarse ingredient comprises a bronchodilator.

16. A bimodal pharmaceutical composition according to Claim 14 **characterised in that** the anti-inflammatory agent is a corticosteroid.

17. A bimodal pharmaceutical composition according to Claim 16 **characterised in that** the corticosteroid is selected from one or more of beclomethasone, fluticasone, budesonide, flunisolide, ciclesonide, triamcinolone, and mometasone, and pharmaceutically acceptable esters thereof.

18. A bimodal pharmaceutical composition according to Claim 15 **characterised in that** the bronchodilator is selected from β2-agonists, non-selective β-stimulants, xanthine bronchodilators, anti-chloinergics, isomers and/or combinations thereof.

19. A bimodal pharmaceutical composition according to claim 15 **characterised in that** the composition comprises a combination of fluticasone, or a pharmaceutically acceptable ester thereof, and formoterol, or a pharmaceutically acceptable salt thereof.

20. A pharmaceutical composition according to Claim 6 **characterised in that** at least one of the active ingredients is systemically active in a patient.

21. A bimodal pharmaceutical composition according to Claim 20 **characterised in that** the fine active ingredient is systemically active in a patient.

22. A bimodal pharmaceutical composition according to Claim 21 **characterised in that** the fine ingredient is selected from one or more of, an antibiotic and a macromolecular medicament.

23. A bimodal pharmaceutical composition according to claims 20 to 22 **characterised in that** the fine ingredient is delivered to the respiratory tract.

24. A bimodal pharmaceutical composition according to Claim 22 **characterised in that** the large macromolecule is selected from one or more polypeptides.

25. A bimodal pharmaceutical composition according to Claim 24 **characterised in that** the macromolecule is selected from insulin, growth hormone, leuprolide, interferon and parathyroid hormone.

26. A bimodal pharmaceutical composition according to Claim 22 **characterised in that** the macromolecular medicament is an analgesic compound.

27. A bimodal pharmaceutical composition according to Claim 26 **characterised in that** the analgesic compound is selected from morphine, M6G and fentanyl.

28. A bimodal pharmaceutical composition according to any one of Claims 19 to 27 **characterised in that** the composition includes an absorption enhancer.

29. A bimodal pharmaceutical formulation according to any one of the preceding claims suitable for administration by way of a pressurised aerosol comprising such a pharmaceutical composition in admixture with at least a suitable propellant.

30. A bimodal pharmaceutical composition according to any one of claims 1 to 27 suitable for administration by a dry powder inhaler comprising such a pharmaceutical composition.

31. A bimodal pharmaceutical composition according to Claim 30 **characterised in that** the composition includes a pharmaceutically acceptable adjuvant, diluent or carrier.

32. A bimodal pharmaceutical formulation according to Claim 31 **characterised in that** the pharmaceutical composition to carrier ratio is from 0.01 :1 to 50: 1.

33. A dry powder inhaler containing (i) a particulate coarse active ingredient and (ii) a particulate fine active ingredient, **characterised in that** the coarse ingredient possesses a greater mass median aerodynamic diameter (MMAD) than the fine ingredient, the MMAD of the coarse active ingredient being 4-20µm and the MMAD of the fine active ingredient being 1-4µm, and wherein the coarse ingredient comprises an agent which is active in the central/upper airways of a patient, which ingredients may be administered simultaneously, sequentially or separately.

34. A bimodal pharmaceutical composition according to Claim 13 **characterised in that** a single dosage administrable to a patient is in the range of from 1 µg to 300mg.

35. A bimodal pharmaceutical composition according to Claim 13 **characterised in that** a single dosage administrable to a patient comprises from 3 to 200 µg of the coarse fraction and from 20 to 1,000 µg of the fine fraction.

36. A bimodal pharmaceutical composition according to any one of claims 1 to 30 suitable for administration by way of a nebuliser comprising a suspension of a pharmaceutical composition.

37. A bimodal pharmaceutical composition according to claim 36 **characterised in that** the dosage administered is in the range of from 1 µg to 500 mg.

38. Use of (i) a particulate coarse active ingredient and (ii) a particulate fine active ingredient in the manufacture of a medicament for the treatment of a respiratory disorder, **characterised in that** the coarse ingredient possesses a greater mass median aerodynamic diameter (MMAD) than the fine ingredient, the MMAD of the coarse active ingredient being 4-20µm and the MMAD of the fine active ingredient being 1-4µm, and wherein the coarse ingredient comprises an agent which is active in the central/upper airways of a patient.

39. Use according to Claim 38 **characterised in that** the coarse and fine ingredients are administered as a single composition.

40. Use according to any one of Claims 38 to 40 **characterised in that** the coarse ingredient includes a signalling agent.

41. Use according to any one of claims 38 to 40 wherein the disorder is COPD.

42. Use according to any one of claims 38 to 41 **characterised in that** the fine ingredient is an anti-inflammatory agent.

43. Use according to any one of claims 38 to 42 **characterised in that** the coarse ingredient is a bronchodilator.

44. Use according to claim 42 wherein the anti-inflammatory agent is a corticosteroid.

45. Use of (i) a particulate coarse active ingredient and (ii) a particulate fine active ingredient in the manufacture of a medicament for the treatment of a systemic disorder, **characterised in that** the coarse ingredient possesses a greater mass median aerodynamic diameter (MMAD) than the fine ingredient, the MMAD of the coarse active ingredient being 4-20µm and the MMAD of the fine active ingredient being 1-4µm, and wherein the coarse ingredient comprises an agent which is active in the central/upper airways of a patient.

46. Use according to claim 45, **characterised in that** the composition is administered by inhalation.

47. A bimodal pharmaceutical composition according to Claim 16 **characterised in that** the ratio of bronchodilator to anti-inflammatory agent is within the range from 1: 0.4 to 1 : 167.

48. A process for the manufacture of a bimodal pharmaceutical composition according to Claim 1 which comprises mixing a coarse active ingredient with a fine active ingredient, and optionally at the same time or sequentially mixing a pharmaceutically acceptable adjuvant, diluent or carrier.

## Patentansprüche

1. Bimodale pharmazeutische Zusammensetzung, die wirksame Mengen (i) eines partikulären groben wirksamen Bestandteils und (ii) eines partikulären feinen wirksamen Bestandteils umfasst, **dadurch gekennzeichnet, dass** der grobe Bestandteil einen größeren massemedianen aerodynamischen Durchmesser (MMAD) als der feine Bestandteil besitzt, wobei der MMAD des groben wirksamen Bestandteils 4-20 µm beträgt und der MMAD des feinen wirksamen Bestandteils 1-4 µm beträgt und wobei der grobe Bestandteil ein Agens umfasst, das in den zentralen/oberen Atemwegen eines Patienten wirkt.

2. Bimodale pharmazeutische Zusammensetzung nach Anspruch 1, **dadurch gekennzeichnet, dass** der feine wirksame Bestandteil ein Agens umfasst, das in der Lungenperipherie wirkt.

3. Bimodale pharmazeutische Zusammensetzung nach einem der Ansprüche 1 und 2, **dadurch gekennzeichnet, dass** die aerodynamische Partikelgröße des groben Bestandteils 4 bis 20 µm beträgt.

4. Bimodale pharmazeutische Zusammensetzung nach Anspruch 3, **dadurch gekennzeichnet, dass** mindestens 50 Masse% der groben Partikel eine aerodynamische Partikelgröße von 4 bis 12 µm aufweisen.

5. Bimodale pharmazeutische Zusammensetzung nach Anspruch 4, **dadurch gekennzeichnet, dass** mindestens 50 Masse% der groben Partikel eine aerodynamische Partikelgröße von 6 µm aufweisen.

6. Bimodale pharmazeutische Zusammensetzung nach einem vorangehenden Anspruch, **dadurch gekennzeichnet, dass** die aerodynamische Partikelgröße des feinen Bestandteils 1 bis 4 µm beträgt.

7. Bimodale pharmazeutische Zusammensetzung nach Anspruch 6, **dadurch gekennzeichnet, dass** mindestens 50 Masse% der feinen Partikel eine aerodynamische Partikelgröße von 1 bis 4 µm aufweisen.

8. Bimodale pharmazeutische Zusammensetzung nach Anspruch 7, **dadurch gekennzeichnet, dass** mindestens 50 Masse% der feinen Partikel eine aerodynamische Partikelgröße von 1 µm aufweisen.

9. Bimodale pharmazeutische Zusammensetzung nach einem der vorangehenden Ansprüche, **dadurch gekennzeichnet, dass** sich die pharmazeutische Zusammensetzung zur Behandlung einer oder mehrerer Erkrankung(en), ausgewählt aus Allergie, Anaphylaxie, Arteriitis, Kollagenose, Blutkrankheiten, kardiovaskuläre Erkrankungen, gastrointestinale Erkrankungen, Hyperkalzämie, Muskelerkrankungen, Augenkrankheiten, Nierenerkrankungen, Atemwegserkrankung, rheumatische Erkrankungen und Hautkrankheiten, eignet.

10. Bimodale Zusammensetzung nach einem der vorangehenden Ansprüche, **dadurch gekennzeichnet, dass** die Zusammensetzung ein Signalisierungsmittel einschließt.

11. Bimodale Zusammensetzung nach Anspruch 10, **dadurch gekennzeichnet, dass** das Signalisierungsmittel in dem groben Bestandteil inbegriffen ist.

12. Bimodale Zusammensetzung nach Anspruch 11, **dadurch gekennzeichnet, dass** das grobe Signalisierungsmittel eine trimodale Zusammensetzung schafft.

13. Bimodale pharmazeutische Zusammensetzung nach Anspruch 9, **dadurch gekennzeichnet, dass** sich die pharmazeutische Zusammensetzung zur Behandlung von Atemwegserkrankungen eignet.

14. Bimodale pharmazeutische Zusammensetzung nach Anspruch 13, **dadurch gekennzeichnet, dass** der feine Bestandteil ein entzündungshemmendes Mittel umfasst.

15. Bimodale pharmazeutische Zusammensetzung nach Anspruch 13 oder Anspruch 14, **dadurch gekennzeichnet, dass** der grobe Bestandteil einen Bronchodilatator umfasst.

16. Bimodale pharmazeutische Zusammensetzung nach Anspruch 14, **dadurch gekennzeichnet, dass** das entzündungshemmende Mittel ein Corticosteroid ist.

17. Bimodale pharmazeutische Zusammensetzung nach Anspruch 16, **dadurch gekennzeichnet, dass** das Corticosteroid ausgewählt ist aus einem oder mehreren von Beclomethason, Fluticason, Budesonid, Flunisolid, Ciclesonid, Triamcinolon und Mometason und pharmazeutisch akzeptablen Estern davon.

18. Bimodale pharmazeutische Zusammensetzung nach Anspruch 15, **dadurch gekennzeichnet, dass** der Bronchodilatator ausgewählt ist aus β2-Agonisten, nichtselektiven β-Stimulatoren, Xanthin-Bronchodilatatoren, Anticholinergika, Isomeren und/oder Kombinationen davon.

19. Bimodale pharmazeutische Zusammensetzung nach Anspruch 15, **dadurch gekennzeichnet, dass** die Zusammensetzung eine Kombination von Fluticason oder eines pharmazeutisch akzeptablen Esters davon und Formoterol oder eines pharmazeutisch akzeptablen Salzes davon umfasst.

20. Pharmazeutische Zusammensetzung nach Anspruch 6, **dadurch gekennzeichnet, dass** mindestens einer der wirksamen Bestandteile systemisch in einem Patienten wirkt.

21. Bimodale pharmazeutische Zusammensetzung nach Anspruch 20, **dadurch gekennzeichnet, dass** der feine wirksame Bestandteil systemisch in einem Patienten wirkt.

22. Bimodale pharmazeutische Zusammensetzung nach Anspruch 21, **dadurch gekennzeichnet, dass** der feine Bestandteil ausgewählt ist aus einem oder mehreren von einem Antibiotikum und einem makromolekularen Medikament.

23. Bimodale pharmazeutische Zusammensetzung nach den Ansprüchen 20 bis 22, **dadurch gekennzeichnet, dass** der feine Bestandteil den Atemwegen zugeführt wird.

24. Bimodale pharmazeutische Zusammensetzung nach Anspruch 22, **dadurch gekennzeichnet, dass** das große Makromolekül aus einem oder mehreren Polypeptid(en) ausgewählt ist.

25. Bimodale pharmazeutische Zusammensetzung nach Anspruch 24, **dadurch gekennzeichnet, dass** das Makromolekül ausgewählt ist aus Insulin, Wachstumshormon, Leuprolid, Interferon und Parathyroidhormon.

26. Bimodale pharmazeutische Zusammensetzung nach Anspruch 22, **dadurch gekennzeichnet, dass** das makromolekulare Medikament eine schmerzstillende Verbindung ist.

27. Bimodale pharmazeutische Zusammensetzung nach Anspruch 26, **dadurch gekennzeichnet, dass** die schmerzstillende Verbindung ausgewählt ist aus Morphin, M6G und Fentanyl.

28. Bimodale pharmazeutische Zusammensetzung nach einem der Ansprüche 19 bis 27, **dadurch gekennzeichnet, dass** die Zusammensetzung einen Absorptionsverstärker einschließt.

29. Bimodale pharmazeutische Zusammensetzung nach einem der vorangehenden Ansprüche, die sich zur Verabreichung mittels eines unter Druck stehenden Aerosols eignet, das eine solche pharmazeutische Zusammensetzung unter Beimischung mindestens eines geeigneten Treibmittels umfasst.

30. Bimodale pharmazeutische Zusammensetzung nach einem der Ansprüche 1 bis 27, das sich zur Verabreichung mittels eines Trockenpulverinhalators eignet, der eine solche pharmazeutische Zusammensetzung umfasst.

31. Bimodale pharmazeutische Zusammensetzung nach Anspruch 30, **dadurch gekennzeichnet, dass** die Zusammensetzung einen pharmazeutisch akzeptablen Hilfsstoff, Streckstoff oder Träger einschließt.

32. Bimodale pharmazeutische Zusammensetzung nach Anspruch 31, **dadurch gekennzeichnet, dass** das Verhältnis von pharmazeutischer Zusammensetzung zu Träger 0,01 : 1 bis 50 : 1 beträgt.

33. Trockenpulverinhalator, der (i) einen partikulären groben wirksamen Bestandteil und (ii) einen partikulären feinen wirksamen Bestandteil enthält, **dadurch gekennzeichnet, dass** der grobe Bestandteil einen größeren massemedianen aerodynamischen Durchmesser (MMAD) als der feine Bestandteil besitzt, wobei der MMAD des groben wirksamen Bestandteils 4-20 µm beträgt und der MMAD des feinen wirksamen Bestandteils 1-4 µm beträgt und wobei der grobe Bestandteil ein Agens umfasst, das in den zentralen/oberen Atemwegen eines Patienten wirkt, wobei die Bestandteile gleichzeitig, nacheinander oder separat verabreicht werden können.

34. Bimodale pharmazeutische Zusammensetzung nach Anspruch 13, **dadurch gekennzeichnet, dass** eine einem Patienten verabreichbare Einzeldosis im Bereich von 1 µg bis 300 mg liegt.

35. Bimodale pharmazeutische Zusammensetzung nach Anspruch 13, **dadurch gekennzeichnet, dass** eine einem Patienten verabreichbare Einzeldosis 3 bis 200 µg der groben Fraktion und 20 bis 1.000 µg der feinen Fraktion umfasst.

36. Bimodale pharmazeutische Zusammensetzung nach einem der Ansprüche 1 bis 30, die sich zur Verabreichung mittels eines Zerstäubers eignet, der eine Suspension einer pharmazeutischen Zusammensetzung umfasst.

37. Bimodale pharmazeutische Zusammensetzung nach Anspruch 36, **dadurch gekennzeichnet, dass** die verabreichte Dosis im Bereich von 1 µg bis 500 mg liegt.

38. Verwendung (i) eines partikulären groben wirksamen Bestandteils und (ii) eines partikulären feinen wirksamen Bestandteils bei der Herstellung eines Medikaments zur Behandlung einer Atemwegserkrankung, **dadurch gekennzeichnet, dass** der grobe Bestandteil einen größeren massemedianen aerodynamischen Durchmesser (MMAD) als der feine Bestandteil besitzt, wobei der MMAD des groben wirksamen Bestandteils 4-20 µm beträgt und der MMAD des feinen wirksamen Bestandteils 1-4 µm beträgt und wobei der grobe Bestandteil ein Agens umfasst, das in den zentralen/oberen Atemwegen eines Patienten wirkt.

39. Verwendung nach Anspruch 38, **dadurch gekennzeichnet, dass** der grobe und feine Bestandteil als eine einzige Zusammensetzung verabreicht werden.

40. Verwendung nach einem der Ansprüche 38 bis 40, **dadurch gekennzeichnet, dass** der grobe Bestandteil ein Signalisierungsmittel einschließt.

41. Verwendung nach einem der Ansprüche 38 bis 40, wobei die Erkrankung die chronische obstruktive Lungenerkrankung (COPD) ist.

42. Verwendung nach einem der Ansprüche 38 bis 41, **dadurch gekennzeichnet, dass** der feine Bestandteil ein entzündungshemmendes Mittel ist.

43. Verwendung nach einem der Ansprüche 38 bis 42, **dadurch gekennzeichnet, dass** der grobe Bestandteil ein Bronchodilatator ist.

44. Verwendung nach Anspruch 42, wobei das entzündungshemmende Mittel ein Corticosteroid ist.

45. Verwendung (i) eines partikulären groben wirksamen Bestandteils und (ii) eines partikulären feinen wirksamen Bestandteils bei der Herstellung eines Medikaments zur Behandlung einer systemischen Erkrankung, **dadurch gekennzeichnet, dass** der grobe Bestandteil einen größeren massemedianen aerodynamischen Durchmesser (MMAD) als der feine Bestandteil besitzt, wobei der MMAD des groben wirksamen Bestandteils 4-20 µm beträgt und der MMAD des feinen wirksamen Bestandteils 1-4 µm beträgt und wobei der grobe Bestandteil ein Agens umfasst, das in den zentralen/oberen Atemwegen eines Patienten wirkt.

46. Verwendung nach Anspruch 45, **dadurch gekennzeichnet, dass** die Zusammensetzung durch Inhalation verabreicht wird.

47. Bimodale pharmazeutische Zusammensetzung nach Anspruch 16, **dadurch gekennzeichnet, dass** das Verhältnis von Bronchodilatator zu entzündungshemmendem Mittel im Bereich von 1 : 0,4 bis 1 : 167 liegt.

48. Verfahren zur Herstellung einer bimodalen pharmazeutischen Zusammensetzung nach Anspruch 1, das das Vermischen eines groben wirksamen Bestandteils mit einem feinen wirksamen Bestandteil und optional gleichzeitig oder nachfolgend das Beimischen eines pharmazeutisch akzeptablen Hilfsstoffs, Streckstoffs oder Trägers umfasst.

## Revendications

1. Composition pharmaceutique bimodale comprenant des quantités efficaces de (i) un ingrédient actif particulaire grossier et (ii) un ingrédient actif particulaire fin, **caractérisée en ce que** l'ingrédient grossier possède un diamètre aérodynamique médian en masse (MMAD) supérieur à celui de l'ingrédient fin, le MMAD de l'ingrédient actif grossier étant de 4 à 20 µm et le MMAD de l'ingrédient actif fin étant de 1 à 4 µm, et dans laquelle l'ingrédient grossier comprend un agent qui est actif dans les voies respiratoires centrales/supérieures d'un patient.

2. Composition pharmaceutique bimodale selon la revendication 1, **caractérisée en ce que** l'ingrédient actif fin comprend un agent qui est actif dans la périphérie du poumon.

3. Composition pharmaceutique bimodale selon l'une quelconque des revendications 1 et 2, **caractérisée en ce que** la taille aérodynamique de particule de l'ingrédient grossier est de 4 à 20 µm.

4. Composition pharmaceutique bimodale selon la revendication 3, **caractérisée en ce qu'**au moins 50 % p/p des particules grossières ont une taille aérodynamique de particule de 4 à 12 µm.

5. Composition pharmaceutique bimodale selon la revendication 4, **caractérisée en ce qu'**au moins 50 % p/p des particules grossières ont une taille aérodynamique de particule de 6 µm.

6. Composition pharmaceutique bimodale selon l'une quelconque des revendications précédentes, **caractérisée en ce que** la taille aérodynamique de particule de l'ingrédient fin est de 1 à 4 µm.

7. Composition pharmaceutique bimodale selon la revendication 6, **caractérisée en ce qu'**au moins 50 % p/p des particules fines ont une taille aérodynamique de particule de 1 à 4 µm.

8. Composition pharmaceutique bimodale selon la revendication 7, **caractérisée en ce qu'**au moins 50% p/p des particules fines ont une taille aérodynamique de particule de 1 µm.

9. Composition pharmaceutique bimodale selon l'une quelconque des revendications précédentes, **caractérisée en ce que** la composition pharmaceutique est appropriée pour le traitement d'un ou plusieurs troubles choisis parmi une allergie, l'anaphylaxie, l'artérite, la maladie du collagène, les troubles sanguins, les troubles cardiovasculaires, les troubles gastro-intestinaux, l'hypercalcémie, les troubles musculaires, les troubles oculaires, les troubles rénaux, une maladie respiratoire, les troubles rhumatismaux et les troubles de la peau.

10. Composition bimodale selon l'une quelconque des revendications précédentes, **caractérisée en ce que** la composition comprend un agent de signalisation.

11. Composition bimodale selon la revendication 10, **caractérisée en ce que** l'agent de signalisation est compris dans l'ingrédient grossier.

12. Composition bimodale selon la revendication 11, **caractérisée en ce que** l'agent de signalisation grossier crée une composition trimodale.

13. Composition pharmaceutique bimodale selon la revendication 9, **caractérisée en ce que** la composition pharmaceutique est appropriée pour le traitement de troubles respiratoires.

14. Composition pharmaceutique bimodale selon la revendication 13, **caractérisée en ce que** l'ingrédient fin comprend un agent anti-inflammatoire.

15. Composition pharmaceutique bimodale selon la revendication 13 ou la revendication 14, **caractérisée en ce que** l'ingrédient grossier comprend un bronchodilatateur.

16. Composition pharmaceutique bimodale selon la revendication 14, **caractérisée en ce que** l'agent anti-inflammatoire est un corticostéroïde.

17. Composition pharmaceutique bimodale selon la revendication 16, **caractérisée en ce que** le corticostéroïde est choisi parmi un ou plusieurs de la béclométhasone, la fluticasone, le budésonide, le flunisolide, le ciclésonide, la triamcinolone, et la mométasone, et les esters pharmaceutiquement acceptables de ceux-ci.

18. Composition pharmaceutique bimodale selon la revendication 15, **caractérisée en ce que** le bronchodilatateur est choisi parmi les agonistes β2, les β-stimulants non sélectifs, les bronchodilatateurs xanthine, les anticholinergiques, les isomères et/ou les combinaisons de ceux-ci.

19. Composition pharmaceutique bimodale selon la revendication 15, **caractérisée en ce que** la composition comprend une combinaison de fluticasone, ou d'un ester pharmaceutiquement acceptable de celle-ci, et de formotérol, ou d'un sel pharmaceutiquement acceptable de celui-ci.

20. Composition pharmaceutique selon la revendication 6, **caractérisée en ce qu'**au moins l'un des ingrédients actifs est actif de manière systémique chez un patient.

21. Composition pharmaceutique bimodale selon la revendication 20, **caractérisée en ce que** l'ingrédient actif fin est actif de manière systémique chez un patient.

22. Composition pharmaceutique bimodale selon la revendication 21, **caractérisée en ce que** l'ingrédient fin est choisi parmi un ou plusieurs d'un antibiotique et d'un médicament macromoléculaire.

23. Composition pharmaceutique bimodale selon les revendications 20 à 22, **caractérisée en ce que** l'ingrédient fin est administré aux voies respiratoires.

24. Composition pharmaceutique bimodale selon la revendication 22, **caractérisée en ce que** la grande macromolécule est choisie parmi un ou plusieurs poly(peptides).

25. Composition pharmaceutique bimodale selon la revendication 24, **caractérisée en ce que** la macromolécule est choisie parmi l'insuline, l'hormone de croissance, le leuprolide, l'interféron et la parathormone.

26. Composition pharmaceutique bimodale selon la revendication 22, **caractérisée en ce que** le médicament macromoléculaire est un composé analgésique.

27. Composition pharmaceutique bimodale selon la revendication 26, **caractérisée en ce que** le composé analgésique est choisi parmi la morphine, le M6G et le fentanyle.

28. Composition pharmaceutique bimodale selon l'une quelconque des revendications 19 à 27, **caractérisée en ce que** la composition comprend un amplificateur d'absorption.

29. Formulation pharmaceutique bimodale selon l'une quelconque des revendications précédentes, appropriée pour une administration à l'aide d'un aérosol sous pression comprenant une telle composition pharmaceutique en mélange avec au moins un propulseur approprié.

30. Composition pharmaceutique bimodale selon l'une quelconque des revendications 1 à 27, appropriée pour une administration par un inhalateur de poudre sèche comprenant une telle composition pharmaceutique.

31. Composition pharmaceutique bimodale selon la revendication 30, **caractérisée en ce que** la composition comprend un adjuvant, diluant ou véhicule pharmaceutiquement acceptable.

32. Formulation pharmaceutique bimodale selon la revendication 31, **caractérisée en ce que** le rapport composition pharmaceutique sur véhicule est de 0,01 : 1 à 50 : 1.

33. Inhalateur de poudre sèche contenant (i) un ingrédient actif particulaire grossier et (ii) un ingrédient actif particulaire fin, **caractérisé en ce que** l'ingrédient grossier possède un diamètre aérodynamique médian en masse (MMAD) supérieur à celui de l'ingrédient fin, le MMAD de l'ingrédient actif grossier étant de 4 à 20 µm et le MMAD de l'ingrédient actif fin étant de 1 à 4 µm, et dans lequel l'ingrédient grossier comprend un agent qui est actif dans les voies respiratoires centrales/supérieures d'un patient, lesquels ingrédients peuvent être administrés simultanément, séquentiellement ou séparément.

34. Composition pharmaceutique bimodale selon la revendication 13, **caractérisée en ce qu'**une posologie unique pouvant être administrée à un patient est dans la gamme de 1 µg à 300 mg.

35. Composition pharmaceutique bimodale selon la revendication 13, **caractérisée en ce qu'**une posologie unique pouvant être administrée à un patient comprend de 3 à 200 µg de la fraction grossière et de 20 à 1 000 µg de la fraction fine.

36. Composition pharmaceutique bimodale selon l'une quelconque des revendications 1 à 30, appropriée pour une administration à l'aide d'un nébuliseur comprenant une suspension d'une composition pharmaceutique.

37. Composition pharmaceutique bimodale selon la revendication 36, **caractérisée en ce que** la posologie administrée est dans la gamme de 1 µg à 500 mg.

38. Utilisation (i) d'un ingrédient actif particulaire grossier et (ii) un ingrédient actif particulaire fin dans la fabrication d'un médicament pour le traitement d'un trouble respiratoire, **caractérisée en ce que** l'ingrédient grossier possède un diamètre aérodynamique médian en masse (MMAD) supérieur à celui de l'ingrédient fin, le MMAD de l'ingrédient actif grossier étant de 4 à 20 µm et le MMAD de l'ingrédient actif fin étant de 1 à 4 µm, et dans laquelle l'ingrédient grossier comprend un agent qui est actif dans les voies respiratoires centrales/supérieures d'un patient.

39. Utilisation selon la revendication 38, **caractérisée en ce que** les ingrédients grossiers et fins sont administrés sous forme de composition unique.

40. Utilisation selon l'une quelconque des revendications 38 à 40, **caractérisée en ce que** l'ingrédient grossier comprend un agent de signalisation.

41. Utilisation selon l'une quelconque des revendications 38 à 40, dans laquelle le trouble est une COPD (broncho-pneumopathie chronique obstructive).

42. Utilisation selon l'une quelconque des revendications 38 à 41, **caractérisée en ce que** l'ingrédient fin est un agent anti-inflammatoire.

43. Utilisation selon l'une quelconque des revendications 38 à 42, **caractérisée en ce que** l'ingrédient grossier est un bronchodilatateur.

44. Utilisation selon la revendication 42, dans laquelle l'agent anti-inflammatoire est un corticostéroïde.

45. Utilisation de (i) un ingrédient actif particulaire grossier et (ii) un ingrédient actif particulaire fin dans la fabrication d'un médicament pour le traitement d'un trouble systémique, **caractérisée en ce que** l'ingrédient grossier possède un diamètre aérodynamique médian en masse (MMAD) supérieur à celui de l'ingrédient fin, le MMAD de l'ingrédient actif grossier étant de 4 à 20 µm et le MMAD de l'ingrédient actif fin étant de 1 à 4 µm, et dans laquelle l'ingrédient grossier comprend un agent qui est actif dans les voies respiratoires centrales/supérieures d'un patient.

46. Utilisation selon la revendication 45, **caractérisée en ce que** la composition est administrée par inhalation.

47. Composition pharmaceutique bimodale selon la revendication 16, **caractérisée en ce que** le rapport de bronchodilatateur sur agent anti-inflammatoire est dans la gamme de 1 : 0,4 à 1:167.

48. Procédé pour la fabrication d'une composition pharmaceutique bimodale selon la revendication 1, qui comprend le mélange d'un ingrédient actif grossier à un ingrédient actif fin, et facultativement dans le même temps ou séquentiellement le mélange d'un adjuvant, diluant ou véhicule pharmaceutiquement acceptable.
